# EUROPEAN PATENT APPLICATION

(11) **EP 2 471 522 A1**
(43) Date of publication of application: **04.07.2012**
(21) Application number: 12158073.2
(22) Date of filing: 24.04.2008
(51) Int. Cl.: A61K 9/48, A61K 31/196

(54) **Oral dosage form providing fast absorption of drug**

(30) Priority: 24.05.2007 EP 07108861
(62) Divisional of application: 08749697.2
(71) Applicant: Novartis AG, 4056 Basel (CH)
(72) Inventor: Shaw, Lance, 1260 Nyon (CH); Kienzler, Jean-Luc, 01210 Ornex (FR); Rault, Isabelle, 01170 Segny (FR)
(74) Representative: Liphardt, Bernd

(57) **Abstract**

The invention relates to a certain oral dosage form providing absorption of the drug diclofenac in an ultrafast manner.

## Description

The present invention relates to oral administration of the drug diclofenac which has been widely used as, inter alia, an anti-inflammatory agent or for the treatment of pain.

For the treatment of pain, an obvious goal is to have a rapid onset of action for an as early a relief as possible of the patient. It is accepted that onset of action is directly related to rate of absorption.

In the art, the rate of absorption is usually described by the use of the pharmacokinetic parameters "time to peak plasma concentration" (tₘₐₓ) and "peak plasma concentration" (Cₘₐₓ). However, these parameters are limited in their ability to assess rate of absorption and the Federal Drug Administration in the USA have introduced a new criterion to be used to assess early exposure. An early exposure measure may be indicative of the clinical onset of action. This is called the AUC_{tmaxref} which is defined as the area under the pharmacokinetic curve (AUC) until the median tₘₐₓ for the reference formulation. This measure of early systemic exposure, together with peak exposure (Cₘₐₓ) will better assess the rate and extent of absorption.

It is further known that the fundamental processes controlling drug absorption are drug dissolution and gastrointestinal permeability. As a consequence it is also known that the physicochemical characteristics of the drug and/or the dosage form can affect the rate of absorption and therefore onset of action. For drugs which have low solubilities and dissolution rates such as ibuprofen and diclofenac - classified as so-called Class II compounds according to the "Biopharmaceutics Classification System" - it is known that the rate of absorption is limited by the dissolution rate of the drug. It is very well known in the art that for drugs with poor solubility and dissolution rates the use of a salt form of the drug acts to buffer the solution and modify the microenvironment which act to increase the solubility and dissolution rate respectively, leading to an increased absorption rate.

Relevant examples from the art include pharmacokinetic studies where it was demonstrated that an ibuprofen salt - ibuprofen lysinate - is more rapidly absorbed than ibuprofen administered as the free acid.

It is also known in the art that a soft gelatin capsule containing dissolved ibuprofen has fast absorption (tₘₐₓ=0.54 h), as does a tablet comprising ibuprofen lysinate (tₘₐₓ=0.54 h), both being faster than a tablet comprising ibuprofen (tₘₐₓ=1.3 h), in a pharmacokinetic study with the drug dosed at 400mg.

It is also known in the art that for ibuprofen the sodium dihydrate salt (tₘₐₓ=0.6 h) has the same fast absorption when compared to tablets comprising ibuprofen lysinate salt (tₘₐₓ=0.7 h) and ibuprofen soft gelatin capsules comprising dissolved ibuprofen (tₘₐₓ=0.7 h), all much faster than tablets comprising ibuprofen (tₘₐₓ=1.4 h).

Oral dosage forms comprising a salt form of diclofenac such as the potassium salt are known in the art and have fast absorption as observed in a study which showed that a tablet comprising diclofenac potassium salt had a tₘₐₓ of 0.9 h.

An object of the present invention was to find an oral dosage form of diclofenac with substantially faster absorption, i.e. onset of action, than the - already fast absorbed - diclofenac potassium tablets (e.g. Voltaren Dolo®, Cataflam®) mentioned above.

When tentatively applying the "soft gelatin capsule" technology to diclofenac, especially with a soft gelatin capsule comprising dissolved potassium diclofenac, the present inventors expected, an analogy to ibuprofen, that diclofenac salt tablets and soft gelatin capsules should have similar absorption rates. Surprisingly, however, it has been found that when applying (dissolved) diclofenac salts in soft gelatin capsules, the rate of absorption is ultrafast compared with the already fast-acting potassium salt tablets.

The invention therefore relates to the use of a pharmaceutical acceptable diclofenac salt (for the manufacture of an oral medicament) in the form of a soft gelatin capsule for the ultrafast treatment of pain,
wherein said pharmaceutical acceptable diclofenac salt is present in said soft gelatin capsule in solution; and
wherein said ultrafast treatment of pain is defined by an AUC_{tmaxref} value (area under curve until median tₘₐₓ for the reference formulation) which is statistically significantly numerically higher than for an oral dosage form tablet comprising diclofenac potassium at the same dosage [statistical method applied: two one-sided tests procedure (average bioequivalence): analysis of variance of log-transformed AUC_{tmaxref} including terms for sequence, treatment, and period as fixed effects and subject nested within sequence as a random effect].

The statistical method to be applied for assessing AUC_{tmaxref} values is the "two one-sided tests" one (average bioequivalence) described in detail in FDA/CDER Guidances for Industry: Statistical Approaches to Establishing Bioequivalence (Jan 2001); Bioavailability and Bioequivalence Studies for Orally Administered Drug Products-General Considerations (Mar 2003).

In another embodiment of the invention, it relates to the use of a pharmaceutical acceptable diclofenac salt (for the manufacture of an oral medicament) in the form of a soft gelatin capsule for the ultrafast treatment of pain,
wherein said pharmaceutically acceptable diclofenac salt is present in said soft gelatin capsule in solution; and
wherein said ultrafast treatment of pain is defined by an AUC_{tmaxref} value (area under curve until median tₘₐₓ for the reference formulation) of at least 175 ng × hour/mL - preferably at least 180 ng × hour/mL, especially at least 220 ng × hour/mL and in particular at least 250 ng × hour/mL - derived from a pharmacokinetic graph "diclofenac concentration (in the blood) versus time".

For determining AUC_{tmaxref} values, the following criteria do apply: (at least) 42 volunteers, fasted condition, cross-over study, number of sampling points:17, samples taken at time pre-dose, 15 min, 25 min, 35 min, 45 min, 1.0 h, 1.25 h, 1.5 h, 1.75 h, 2.0 h, 2.5 h, 3.0 h, 3.5 h, 4.0 h, 6.0 h, 8.0 h, and 10 h post-dosing.

As to the doses of pharmaceutical acceptable diclofenac salt incorporated into one soft gelatin capsule, these typically are of from 5 up to 100 mg - preferably of from 10 up to 50 mg, especially of from 10 up to 30 mg and in particular of from 10 up to 25 mg.

As to the dosing of the pharmaceutical acceptable diclofenac salt in the disclosed uses, the total dose (administered at the same time, i.e. simultaneously) of said diclofenac salt applied typically is of from 5 up to 100 mg - preferably of from 10 up to 50 mg, especially of from 10 up to 30 mg and in particular of from 10 up to 25 mg. Said total dose may be applied in the form of one or more - preferably one or two, especially two, in another embodiment one - soft gelatin capsule(s).

A total daily dosage above 225 mg per patient typically is not recommended.

The term "pain" is intended to include all forms of pain and ache, e.g.
- mild to moderate acute body pain caused by activity/overuse such as: bumps and bruises, strains and sprains, insect stings and minor cuts, sore muscle, tendonitis, tennis elbow, golfer's elbow or enthesitis
- to moderate persistent body pain caused by degenerative conditions such as: osteoarthritis, chronic back pain, osteoporosis, frozen shoulder, ankylosing spondylitis, carpal tunnel syndrome or Paget's disease
- severe body pain caused by external factors such as lumbago, fractures, post-operative pain, gout attack, cervical spondylosis, rheumatoid arthritis or polymyalgia rheumatic
- mild to moderate acute other pain caused by temporary conditions such as: toothache, sore throat, acute otitis and sinusitis, period pains, migraine, sunburn, herpex simplex or dyspepsia
- mild to moderate persistent other pain such as fibromyalgia, tension headache, parodontitis and gingivitis, diverticulosis, intermittent claudication, thrombophlebitis or shingles
- severe other pain caused by life-threatening conditions such as: heart attack, cancer pain, childbirth, renal colic, abdominal colic, trigeminal neuralgia, sciatica or cluster headache.

The diclofenac soft gelatin capsules manufactured according to present invention are particular beneficial in treating pain indications where a rapid onset of action is of particular importance, e.g. all forms of acute pain such as bumps and bruises, strains and sprains, insect stings and minor cuts, sore muscle, tendonitis, tennis elbow, golfer's elbow, enthesitis, post-operative pain, toothache, sore throat, acute otitis and sinusitis, period pains, headache, migraine, sunburn, herpes simple or dyspepsia.

A pharmaceutical acceptable salt of diclofenac is e.g. diclofenac sodium, diclofenac potassium, diclofenac diethylammonium or diclofenac epolamine. In particular preferred is diclofenac potassium.

A method for manufacturing soft gelatin capsules had been found in the 1930's already, by which a capsule can be produced and filled in a single operation. Thus, the term "soft gelatin capsule" is generally acknowledged and well understood in the field of pharmaceutical technology. Essentially, a soft gelatin capsule comprises a shell of gelatin which is filled with and encloses a liquid. Said liquid, according to the present invention, is preferably a solution (but may also be a suspension) of a pharmaceutically acceptable salt of diclofenac. The solvents used are e.g. oils, such as vegetable, animal or mineral oils, liquid hydrocarbons, volatile oils. polyethylene glycols or mixtures of polyethylene glycols with water. Preferred are mixtures of polyethylene glycols with water. The shell of soft gelatin capsule topically includes a softener, e.g. glycerol, sorbitol, or polyethylene glycols themselves. Preferred is a mixture of sorbitol and at one sorbitane, e.g. "Polysorb 85/70/00" from Roquette Pharma (France). An example for suitable soft gelatin capsules, and some principles of their buildup, composition and manufacture are outlined in US patent 4,744,988 (Brox).

Soft gelatin capsules comprising 12.5 mg and 25 mg of diclofenac potassium have been manufactured in a manner known per se, viz. in close analogy to the "Example" disclosed in US patent 4,744,988, columns 6 and 7 -which means using the same components as disclosed there but replacing diazepam with diclofenac potassium as active substance. In even more detail, said two compositions are as follows.

### Example 1: Composition of 12.5 mg Diclofenac potassium soft gelatin capsule

Composition of the capsule fill contents:

| Name of ingredients | Quantities (mg/ capsule) |
|---|---|
| Drug substance | |
| Diclofenac potassium | 12.50 |

| Other ingredients | |
|---|---|
| Macrogol 600 (= Polyethylene glycol 600) | 100.00 |
| Glycerol 85% | 6.25 |
| Purified water | 6.25 |

Composition of the capsule shell:

| Name of ingredients | Quantities ( mg / capsule) |
|---|---|
| Gelatin | 51.34 |
| Glycerol 85% | 14.93 |
| Sorbitol liquid, partially dehydrogenated (e.g. Polysorb 85/70/00) | 10.86 |
| Quinoline yellow 70% (= E104) | 0.0060 |

### example 2: Composition of 25.0 mg Diclofenac potassium soft gelatin capsule

Composition of the capsule fill contents:

| Name of ingredients | Quantities (mg/ capsule) |
|---|---|
| Drug substance | |
| Diclofenac potassium | 25.0 |

| Other ingredients | |
|---|---|
| Macrogol 600 (= Polyethylene glycol 600) | 200.00 |
| Glycerol 85% | 12.50 |
| Purified water | 12.50 |

Composition of the capsule shell:

| Name of ingredients | Quantities (mg / capsule) |
|---|---|
| Gelatin | 89.85 |
| Glycerol 85% | 26.12 |
| Sorbitol liquid, partially dehydrogenated (e.g. Polysorb 85/70/00) | 19.00 |
| Quinoline yellow 70% (= E104) | 0.011 |

The beneficial properties due to oral administration of diclofenac soft gelatin capsules can be seen e.g. from the following tests:

### (1) Comparative bioavailability study: Comparison of two 12.5 mg diclofenac potassium tablets with two 12.5 mg diclofenac potassium soft gelatin capsules

The following products were compared in a two way cross over single dose pharmacokinetic study using 42 fasted healthy human volunteers:
Test formula A: soft gelatin capsules from example 1
Test formula B: commercially available swallow tablets containing diclofenac potassium 12.5mg

Each volunteer swallowed two samples of one of the formulations equivalent to 25mg of diclofenac potassium, followed by 240 mL of water on two separate occasions at least 14 days apart. Blood samples were taken pre-dose and at 15 min, 25 min, 35 min, 45 min, 1.0 h, 1.25 h, 1.5 h, 1.75 h, 2.0 h, 2.5 h, 3.0 h, 3.5 h, 4.0 h, 6.0 h, 8.0 h, and 10 hours post dose. Serum diclofenac potassium levels were determined by a fully validated LC/MS-MS method and diclofenac potassium plasma levels vs time plots were plotted for each volunteer. The mean AUC_{tmaxref} for formulation A was 294 ng × hour/mL vs 158 ng × hour/mL for formulation B.

| Mean plasma concentration of diclofenac (ng/mL) | | |
|---|---|---|
| Time | Soft gelatin capsule | Tablet |
| Pre-dose | 0.0 | 0.0 |
| 15 min | 385 | 261 |
| 25 min | 915 | 439 |
| 35 min | 764 | 384 |
| 45 min | 477 | 321 |
| 1 h | 256 | 226 |
| 1.25 h | 177 | 179 |
| 1.5 h | 134 | 159 |
| 1.75 h | 103 | 137 |
| 2h | 74 | 108 |
| 2.5 h | 52 | 97 |
| 3h | 38 | 93 |
| 3.5 h | 27 | 64 |
| 4h | 21 | 41 |
| 6h | 8 | 10 |
| 8h | 4 | 4 |
| 10 h | Not estimable | 2 |

### (2) Comparative bioavailability study: Comparison of two 12.5 mg diclofenac potassium tablets with one 25 mg diclofenac potassium soft gelatin capsule

The 25mg Diclofenac potassium soft gelatin capsule was tested in a pharmacokinetic study using a similar design as detailed under (1) for the 12.5mg Diclofenac potassium soft gelatin capsule, except that the soft gelatin capsule was administered as one 25mg dosage form instead of two 12.5mg ones. The ultrafast absorption rate for the 25mg soft gelatin capsule was observed, when compared to two 12.5mg tablets.
(3) Clinical study in 3^{rd} molar extraction: Double-blind, double-dummy, placebo-controlled, parallel-group, randomized trial of diclofenac potassium soft gelatin capsule (12.5 mag) vs paracetamol (500 mg) tablets and placebo in patients with moderate or severe pain within 8 hours of extraction of impacted third molars. All are treated with an initial dose of 2 soft gelatin capsules or 2X 500 mg of paracetamol tablets or placebo followed by 1 or 2 capsules or tablets, as needed, every 4 to 6 hours, up to a maximum of 6 of each type. Efficacy in control of acute pain is determined at baseline, and then at 20 minutes, 40 minutes, 1, 2, 3, 4, 5, and 6 hours after dosing. Significantly higher levels of analgesic are observed in the soft gelatin capsule group at 20 minutes, 40 minutes and 1 hour after the initial dose, compared to paracetamol group.

## Claims

1. Use of a pharmaceutically acceptable diclofenac salt for the manufacture of an oral medicament in the form of a soft gelatin capsule for the ultrafast treatment of pain, wherein said pharmaceutically acceptable diclofenac salt is present in said soft gelatin capsule in solution; and
wherein said ultrafast treatment of pain is defined by an AUC_{fmaxref} value (area under curve until median tₘₐₓ for the reference formulation) which is statistically significantly numerically higher than for an oral dosage form tablet comprising diclofenac potassium at the same dosage [statistical method applied: two one-sided tests procedure (average bioequivatence): analysis of variance of tog-transformed AUC_{tmaxref} including terms for sequence, treatment, and period as fixed effects and subject nested within sequence as a random effect].

2. Use of a pharmaceutical acceptable diclofenac salt for the manufacture of an oral medicament in the form of a soft gelatin capsule for the ultrafast treatment of pain,
wherein said pharmaceutically acceptable diclofenac salt is present in said soft gelatin capsule in solution; and
wherein said ultrafast treatment of pain is defined by an AUC_{tmaxref} value (area under curve until median tₘₐₓ for the reference formulation) of at least 175 ng × hour/mL derived from a pharmacokinetic graph "diclofenac concentration (in the blood) versus time".

3. Use according to claim 2, wherein said ultrafast treatment of pain is defined by an AUC_{tmaxref} value of at least 250 ng × hour/mL derived from a pharmacokinetic graph "diclofenac concentration versus time".

4. Use according to claim 1, wherein the dictofenac salt used is diclofenac potassium.

5. Use according to claim 2 or claim 3, wherein the diclofenac salt used is diclofenac potassium.

6. Use according to claim 1 or claim 4, wherein the dose of said diclofenac salt is of from 5 up to 100 mg per soft gelatin capsule.

7. Use according to claim 1 or claim 4, wherein the dose of said diclofenac salt is of from 10 up to 50 mg per soft gelatin capsule.

8. Use according to anyone of claims 2, 3 or 5, wherein the dose of said dictofenac salt is of from 5 up to 25 mg.

9. Use according to anyone of claim 2, 3 or 5, wherein the dose of said diclofenac salt is of from 15 up to 25 mg.

10. A method of treating pain in mammals including humans in an ultrafast manner, which method comprises orally administering a pharmaceutical acceptable salt of diclofenac in the form of at least one soft gelatin capsule,
wherein said pharmaceutically acceptable salt of diclofenac is present in said at least one soft gelatin capsule in solution; and
wherein said ultrafast treatment of pain is defined by an AUC_{tmaxfef} value (area under curve until median tₘₐₓ for the reference formulation) which is statistically significantly numerically higher than for an oral dosage form tablet comprising diclofenac potassium at the same dosage [statistical method applied: two one-sided tests procedure (average bioequivalence): analysis of variance of log-transformed AUC_{tmaxref} including terms for sequence, treatment, and period as fixed effects and subject nested within sequence as a random effect].

11. A method of treating pain in mammals including humans in an ultrafast manner, which method comprises orally administering a pharmaceutical acceptable salt of diclofenac in the form of one or more soft gelatin capsule(s),
wherein said pharmaceutically acceptable salt of diclofenac is present in said soft gelatin capsule(s) in solution; and,
wherein said treatment of pain in an ultrafast manner is defined by an AUC_{tmaxref} value (area under curve until median tₘₐₓ for the reference formulation) of at least 175 ng × hour/mL derived from a pharmacokinetic graph "diclofenac concentration (in the blood) versus time".

12. The method according to claim 11, wherein the dose of said diclofenac salt is of from 5 up to 25 mg and is applied by administration of one or two soft gelatin capsules at the same time.

13. The method according to claim 11, wherein the dose of said diclofenac salt is of from 15 up to 25 mg and is applied by simultaneous administration of two soft gelatin capsules.
